# EUROPEAN PATENT SPECIFICATION

(11) **EP 2 936 131 B1**
(45) Date of publication and mention of the grant of the patent: **01.02.2017**
(21) Application number: 13826626.7
(22) Date of filing: 18.12.2013
(51) Int. Cl.: G01N 27/07, A61B 10/00, G01N 1/38

(54) **DEVICE FOR MEASURING AMMONIACAL NITROGEN IN LIQUID MANURE, PROCESS THAT USES SAID DEVICE FOR SAID PURPOSES AND KIT THAT CONTAINS IT**
VORRICHTUNG ZUR MESSUNG VON AMMONIUMSTICKSTOFF IN GÜLLE, VERFAHREN UNTER VERWENDUNG DIESER VORRICHTUNG FÜR DEN GENANNTEN ZWECKE UND KIT DAMIT
DISPOSITIF POUR MESURER L'AZOTE AMMONIACAL DANS UN FUMIER LIQUIDE, PROCÉDÉ QUI EMPLOIE LEDIT DISPOSITIF DANS LESDITS OBJECTIFS ET KIT QUI LE CONTIENT

(30) Priority: 21.12.2012 ES 201231995
(43) Date of publication of application: 28.10.2015
(73) Proprietor: Yagüe Carrasco, María Rosa, 46520 Puerto de Sagunto, Valencia (ES)
(72) Inventor: Yagüe Carrasco, María Rosa, 46520 Puerto de Sagunto, Valencia (ES)
(74) Representative: Oficina Ponti, SLP
(86) International application number: PCT/IB2013/061098
(87) International publication number: WO 2014/097183

(56) References cited:
- EP-A2- 0 175 326
- ES-A1- 2 319 369
- US-A1- 2009 193 880
- YAGUEE M R ET AL: "On-farm Measurement of Electrical Conductivity for the Estimation of Ammonium Nitrogen Concentration in Pig Slurry", JOURNAL OF ENVIRONMENTAL QUALITY, AMERICAN SOCIETY OF AGRONOMY, CROP SCIENCE SOCIETY OF AMERICA AND SOIL SCIENCE SOCIETY OF AMERICA, US, vol. 41, no. 3, 1 May 2012 (2012-05-01), pages 893-900, XP008167811, ISSN: 0047-2425, DOI: 10.2134/JEQ2011.0352
- PROVOLO ET AL: "In situ determination of slurry nutrient content by electrical conductivity", BIORESOURCE TECHNOLOGY, ELSEVIER BV, GB, vol. 98, no. 17, 23 August 2007 (2007-08-23), pages 3235-3242, XP022208948, ISSN: 0960-8524, DOI: 10.1016/J.BIORTECH.2006.07.018
- MARA R YAGE ET AL: "Measurement and estimation of the fertiliser value of pig slurry by physicochemical models: Usefulness and constraints", BIOSYSTEMS ENGINEERING, ACADEMIC PRESS, UK, vol. 111, no. 2, 22 November 2011 (2011-11-22), pages 206-216, XP028440452, ISSN: 1537-5110, DOI: 10.1016/J.BIOSYSTEMSENG.2011.11.013 [retrieved on 2011-11-25]

## Description

The present invention relates to a device for performing dilutions, which makes it possible to determine the concentration of ammoniacal nitrogen in liquid manure or slurry. In particular, the invention relates to a device for measuring the concentration of nitrogen in ammonium form in slurry (NH₄⁺-N), whether the origin of the latter is porcine, bovine, duck, poultry or other species, and which has ammoniacal nitrogen.

The present invention also relates to the process for measuring the concentration of ammoniacal nitrogen in liquid manure or slurry from the electrical conductivity, using the device according to the first aspect of the invention, and to the kit that comprises said device.

### Background of the invention

Liquid manure or slurry is primarily used as a fertilizer due to its renowned richness in nutrients. The most habitual criterion for fertilizing is based on the nitrogen content when it is applied with organic fertilizers such as slurry, since this element is the most limiting nutrient for crops.

Nitrogen from slurry may vary depending on the origin of the slurry. Thus, we find pig slurry, cattle slurry, duck slurry, or other slurries, with different concentrations of nitrogen. Pig slurry is characterized by a significant nitrogen concentration (N) in ammonium form (NH₄⁺); this form of mineral nitrogen is available for crops at the time of the application thereof. The concentration of ammoniacal nitrogen in slurry may also vary depending on the age of the animal, the time of storage in the pit, the management of the water in farm, the stirring in pit, etc. This variability in the nitrogen concentration in slurry requires an analysis process and a device designed to determine the nitrogen concentration in a safe, reliable manner.

Therefore, processes and devices designed to analyze the concentration of ammoniacal nitrogen in slurry have been developed in order to determine the quantity of slurry to be applied as a fertilizer in crops depending on the nitrogen concentration thereof. Adding doses of slurry greater than the quantities of nitrogen that the crop can absorb represents excess nitrogen for the environment, whereas adding lower quantities causes deficiencies in the crops, with a loss of yield.

There are different processes and devices for determining the concentration of ammoniacal nitrogen in slurry. The most reliable and precise process is the one used with analytical techniques in the laboratory; however, it is not the most suitable in economic terms or in terms of immediacy for the stockbreeder or farmer. There are devices that may be used *in situ*, i.e. in the fields, which use techniques such as conductimetry, reflectometry, near-infrared spectroscopy (NIR) or others. There are devices that measure the ammoniacal nitrogen in slurry directly and devices that measure it indirectly, for example, from the conductivity. Thus, for example, there is a piston-type device for the taking of samples, and direct-measurement devices from the company "Agros" and the company "Quantofix" which use corrosive reagents.

The piston-type device for the taking of samples has the disadvantage that it is hard to handle, for which reason it requires qualified personnel to take the samples in a precise manner; moreover, it has plugging problems in the case of liquid manure or slurry.

Those devices that are based on reflectometry and near-infrared spectroscopy require calibration prior to using them; furthermore, they are tedious for in-field handling and the quantity of slurry sample taken is very small (5-30 mL), for which reason the representativity of the slurry sample is questionable.

The devices from the commercial brands "Agros" and "Quantofix" require using corrosive reagents in order to determine the nitrogen concentration in slurry. Moreover, they lose effectiveness when they are stored for some time and their optimal, or reliable, operation is at nitrogen concentrations lower than 5 kg NH₄⁺-N/m³, a concentration which is lower than that which pig slurries farms oftentimes present, with values greater than 5 kg NH₄⁺-N/m³.

On the other hand, it is well-known that the direct measurement of the electrical conductivity of the slurry (without dilution) does not require any device in order to prepare the sample to be analyzed, but it is necessary to use high-range conductivity meters (0-50 dS/m), which are expensive and also require prior calibration in order to convert the conductivity value into the ammoniacal-N concentration value.

The state of the art closest to the device of the present invention, as disclosed in Yaguee .M. R. et al: "On-farm Measurement of Electrical Conductivity for the Estimation of Ammonium Nitrogen Concentration in Pig Slurry", Journal of Environmental Quality, ASA CSSA, (41) 3, 2012, 893-900, is a device based on a dilution measurement, adapted so that the stockbreeder or famer may measure, in a quick, simple manner, the concentration of ammonium nitrogen in slurry, which is supplied in a kit. However, the analysis of the nitrogen concentration with said device uses receptacles that are not very precise, such as 100-mL, or 500-mL, measurement precipitation flasks, which undoubtedly translates into a decrease in the reliability of the measurement; moreover, it requires taking 3 measurements: 100 mL of slurry, 400 mL of distilled water and 500 mL of distilled water. In order to perform the analysis of the nitrogen concentration, the following steps are required:
- in the first place, the conductivity meter is calibrated;
- subsequently, said quantity is collected in the slurry with a first, 100-mL precipitation flask;
- 900 mL of distilled water are added in a second precipitation flask;
- in a third, clean container, with a capacity of 1000 mL, the slurry sample and the distilled water sample are added and mixed;
- once the mixing has been performed, 400 mL of the mixture are transferred to a fourth, 500-mL precipitation flask; and
- using the conductivity meter, the conductivity is measured, which is equal to the ammoniacal nitrogen expressed in NH₄⁺-N kg/m³,
- it is multiplied by 1.35 in order to obtain the total nitrogen. This is a 75% approximation of the total-N in pig slurry is ammoniacal-N.

The conductivity meter is used to determine the concentration of ammoniacal nitrogen in pig slurry and the process uses a dilution of 1 part of slurry per 9 parts of distilled water, the electrical conductivity value whereof, in dS/m, numerically coincides with the content of ammoniacal nitrogen in kg/m³.

Said device has the following disadvantages: the measurement is performed with precipitation flasks, for which reason the precision of the volume measurements is very low and, therefore, so is the precision of the concentration of ammoniacal N; moreover, the precision of the measurement of ammoniacal nitrogen is also affected because part of the measurement of the slurry is in the form of residue in the precipitation flask used for said purposes when the sample is transferred to another precipitation flask with a greater volume; it is also tedious, in the fields, to make up to volume the precipitation flasks with slurry and distilled water; in order to perform the mixing, at least one other receptacle not contained in the kit is required; a receptacle with a capacity of 1000 mL is recommended, which does not leave space to adequately mix the 100 mL of pig slurry and the 900 mL of distilled water; the kit contains a conductivity meter; therefore, the farmer, agriculturist or stockbreeder who already has a conductivity meter would not be able to use it and avoid the cost of the conductivity meter; the use and transport of the kit in the fields is tedious, since it further requires complements, such as the receptacle for the mixing, which are not included in the kit; therefore, it is a kit conceived to be used in the laboratory rather than in the field.

For all these reasons, at present farmers and stockbreeders do not have a reliable, safe, easy-to-use device available, which overcomes the disadvantages of the devices in the state of the art.

### Description of the invention

The device designed to measure ammoniacal nitrogen in slurry according to the invention makes it possible to resolve the aforementioned disadvantages and, moreover, it presents other advantages, which will be described below.

In the present invention, the term "dilution" is understood to mean a mixture of slurry in aqueous solution. In the present invention, the terms "liquid manure" and "slurry" have the same meaning.

The present invention provides a device for measuring the concentration of ammoniacal nitrogen (NH₄⁺-N) in slurry in dilution according to claim 1.

The device of the invention makes it possible to use any commercially available conductivity meter, within the range 0 to 10 dS/m, which the farmers, agriculturists or stockbreeders may already have in their farms, for example, for measuring the conductivity of irrigation water or drinking water; this entails economic savings since they do not need to purchase a new conductivity meter.

The device is configured in such a way that the concentration of ammoniacal nitrogen may be measured regardless of the origin of the slurry, such that volumes V1 and V2, of the first receptacle, designed for taking the slurry samples, and of the second receptacle, designed for taking the water samples, respectively, may be modified and adapted to the type of slurry to be analyzed, such that the volume ratio V1/V2 between the two receptacles provides a dilution the conductivity measurement whereof fulfils the equivalence 1 dS/m = 1 kg NH₄⁺-N/m³. Thus, for example, in order to analyze the concentration of ammoniacal nitrogen in pig slurry origin, said first receptacle and said second receptacle are adapted to house a volume V1 and a volume V2 such that the volume ratio V1/V2 is 1 part of slurry / 9 parts of water (the slurry should not be subjected to any chemical treatment which may alter the electrical conductivity thereof). Consequently, the device should not be used when the slurry has previously been chemically-treated and its electrical conductivity been altered.

The scope of protection of the device according to the present invention includes any other volume ratio V1/V2 between said first receptacle, designed to take slurry samples with volume V1, and said second receptacle, designed to take water samples with volume V2, which provides a dilution of slurry in water that fulfils the equivalence 1 dS/m = 1 kg NH₄⁺-N/m³. It is evident for persons skilled in the art that the variation in said ratio V1/V2 entails modifying the size of said first receptacle and said second receptacle.

In the present invention, it is clear that the device according to the first aspect is not limited to determining the ammoniacal nitrogen in slurry and that it also covers determining the concentration of a component of a sample, provided that said sample must be diluted to a pre-determined proportion in order to find out the concentration of one of the components thereof.

It is well-known that the measurement of electrical conductivity is an indirect measurement of the concentration of ions in solution. Slurries present a pH of approximately 7, i.e. a neutral pH, such that the electrical conductivity of slurry or liquid manure is determined by the concentration of the cations and the anions present in the slurry. The predominant cations in slurry are Na⁺, K⁺, Ca²⁺, Mg²⁺, and NH₄⁺, which are in equilibrium with the predominant anions in slurry, which are SO₄²⁻, PO₄²⁻ and Cl⁻. The predominant cation in pig slurry is NH₄⁺, followed by K⁺ (i.e. both cations could be measured in slurry at the appropriate dilution, water:liquid manure, by electrical conductivity).

In general, liquid manure or slurry is a solution which has a high electrical conductivity (i.e. 15-80 dS/m). For high values of electrical conductivity, the relationship between the latter and the concentration of ions in solution is no longer linear, due to the interactions between the charged ions in the solution. For this reason, the slurry is diluted with water, preferably distilled water, which makes it possible to establish a relationship within the linear range.

The device of the invention is configured in such a way that, once the volume ratio V1/V2 has been pre-determined, direct readings of the concentration of ammoniacal nitrogen in the slurry may be taken from the conductivity, using a standard conductivity meter.

Moreover, the design of the device of the invention improves the precision of the volume measurements (V1 and V2), entails higher precision in the taking of samples, which translates into a higher precision when determining the concentration of ammoniacal N, and ease of use by the farmer or agriculturist directly in the fields, which represents a great advantage with respect to the devices in the state of the art. This is undoubtedly a great advantage, because using the device in the fields does not require additional elements, such as extra receptacles for the taking of the slurry or water samples, or for the mixing of the components, or special conductivity meters.

Advantageously, the configuration of the first member and the second member of the device are susceptible to being integrated in a detachable manner, such that they form a single piece.

It is especially advantageous that the device combines in one piece all the parts involved in measuring the ammoniacal nitrogen in slurry. This advantage translates into precision of the measurement (V1 and V2) of the fluids involved, ease of use and practice because each and every one of the necessary elements to prepare the sample for the analysis of the concentration of ammoniacal nitrogen in slurry is contained therein. Moreover, the device may incorporate a pen-type conductivity meter to measure the conductivity of the dilution prepared in the device.

Said second member comprises an outflow opening in the lateral face thereof, such as, for example, a drain, such that said second receptacle has the volume V2 that corresponds to the quantity of water necessary to adequately prepare the dilution to be analyzed. Said outflow opening comprises closure means and is adapted so that it can be attached, by junction means, to means of conduction for the excess water that exits through said outflow opening when said second receptacle with volume V2 is filled with water.

Also advantageously, the device according to the invention makes it possible to mix the slurry and the water in a complete, accurate and safe manner, because the design and configuration thereof ensure the maintenance of the slurry-to-water ratio, preventing the loss of fluid during the preparation of the mixture of slurry and water. Advantageously, the device is equipped with additional space (air) that makes it possible to vigorously stir the slurry and the water, and thus obtain the adequate dilution of slurry in the sample to be analyzed.

With the device according to the first aspect of the invention, distilled water or water with an electrical conductivity (EC) value less than or equal to 2 dS/m may be used. In the event that EC ≤ 2 dS/m, it is necessary to check whether the conductivity of the pre-established water/slurry dilution is equal to or lower than 3 dS/m. In the event that it is equal to or lower than 3 dS/m, it will be necessary to use distilled water, since the lower the concentration of ammoniacal nitrogen in the slurry, the greater the margin of error when using non-distilled water. In the event that water with EC ≤ 2 dS/m is used, since the conductivity of the pre-established water/slurry dilution is greater than 3 dS/m, the electrical conductivity of the water to be used in the dilution will be measured, and said measurement of the electrical conductivity of the water will be subtracted from the conductivity value of the pre-established water/slurry dilution in order to obtain the concentration of ammoniacal nitrogen in the slurry.

According to the device of the invention, said first member comprises a first receptacle designed to take the slurry samples and a closing lid, with the inner lateral face of said lid including means to join said second member in a detachable manner, said first receptacle for the taking of slurry samples being susceptible to being arranged in an inverted position inside the cavity of the second member upon placing the lid.

Moreover, the inner face of said lid is equipped with a cavity configured so as to house said first receptacle for the taking of slurry samples, with said cavity of the lid including means to join said first receptacle for taking slurry samples in a detachable manner.

Said first receptacle for taking slurry samples comprises a substantially elongated handle that extends from the upper edge of the cavity of said receptacle, in order to facilitate the taking of slurry samples. Said cavity in the lid has a conical-trunk shape, and the neck is adjusted in such a way that it is susceptible to being coupled to the first receptacle for taking slurry samples. This configuration of the cavity in the lid with the first receptacle allows for the remains of slurry adhered to the outer surface of the first receptacle, once the latter is submerged in the slurry when taking the slurry samples, not to interfere with the measurement of volume V1, since these remains will be enclosed inside the cavity of the lid when the first receptacle is threaded to the cavity of the lid.

Said substantially elongated handle presents a cross-section with a substantially concave profile, said concave profile presenting a channel where through the slurry may be emptied out.

Said first receptacle for taking slurry samples comprises detachable junction means to the cavity of the lid on the outer face thereof.

Preferably, the junction means between the lid and the second member, and the junction means between the cavity of the lid and the first receptacle are a thread or similar. However, any other junction means that makes it possible to close the different members and receptacles for a given period of time would also be adequate.

The invention also relates to a kit designed to measure the concentration of ammoniacal nitrogen in slurry, which is characterized in that it comprises:
- a device as defined in the attached claims; and
- a sheet of instructions to prepare the mixture to be analyzed, said sheet of instructions substantially including the steps of the process to be followed in order to analyze the concentration of ammoniacal nitrogen in slurry which uses said device.

The kit according to the second aspect of the invention may or may not include a commercially available conductivity meter, preferably within the range between 0 and 10 dS/m.

The invention also relates to the process to be followed in order to analyze the concentration of ammoniacal nitrogen in slurry which uses said device.

In particular, the third aspect of the invention relates to a process for analyzing the concentration of ammoniacal nitrogen in slurry in the device according to claim 13.

Finally, all the elements of the device are cleaned, dried and stored for subsequent usage.

For a better understanding of what has been explained above, some drawings are attached wherein, schematically and solely as non-limiting examples, a practical case of the invention is presented.

### Brief description of the figures

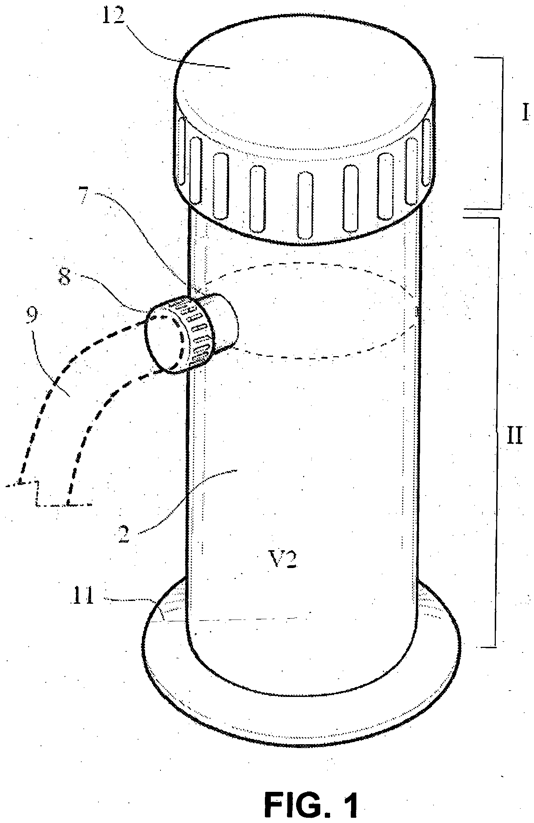

Figure 1 shows a perspective view of the device for performing dilutions between two fluids that makes it possible to measure the concentration of ammoniacal nitrogen in slurry according to the invention, which comprises a first member I and a second member II. Said first member I comprises a first receptacle 1 for taking slurry samples and a lid 12, equipped with a cavity 1a in the inner face thereof, and a second member II, which comprises, on the lateral face thereof, a drain 7 at a height h from the base, which delimits the volume V2 of the second receptacle 2. Said drain may be closed and opened with a closing cap 8. Optionally, an outflow tube 9 may be anchored onto drain 7, such that it is possible to conduct the excess water from the second member II, housing a volume V2 in the second receptacle 2. The configuration of said first member I and said second member II makes it possible to join both members, I and II, in a detachable manner, forming a single piece, as represented in this figure 1. As may be observed in said figure 1, said second member II comprises abase 11 on the lower end thereof designed to improve the stability of the device.

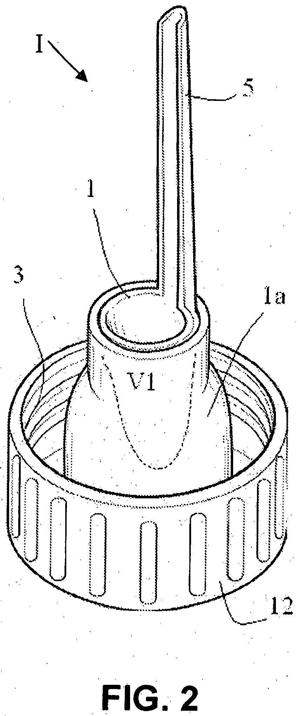

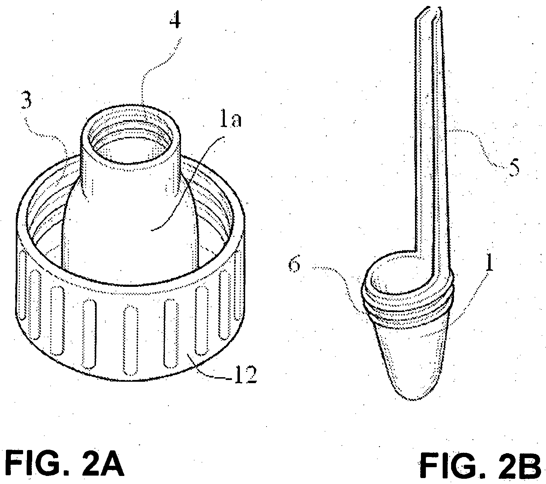

Figure 2 shows a perspective view of the first member I, which comprises the lid 12 equipped with a cavity 1 a that is configured so as to house the first receptacle 1. Said first receptacle 1 presents a cavity with volume V1, and said cavity 1a includes a thread 4 in order to join said first receptacle 1, and a substantially elongated handle 5 that extends from the upper edge of the cavity of said first receptacle 1, where said handle facilitates the taking of slurry samples. Said substantially elongated handle 5 presents a cross-section with a substantially concave profile, said concave profile defining a channel where through the slurry may be emptied out. As may be observed in figure 2, said lid 12 comprises, on the inner lateral face thereof, means 3 designed to join the second member II in a detachable manner (not shown).
Figures 2A and 2B show a breakdown of figure 2.
Figure 2A shows a perspective view of the lid 12 equipped with a cavity 1a with a conical-trunk shape, the inner lateral face of said lid 12 including a thread 3 designed to join said second receptacle 2 in a detachable manner (not shown). The opposite base of said cavity 1a, the neck of the trunk, comprises, on the inner lateral face thereof, a thread 4 designed to join said first receptacle 1 in a detachable manner (not shown).
Figure 2B shows a perspective view of the first receptacle 1 for taking slurry samples. Said first receptacle 1 has a cylindrical shape with a concave-trapezoidal end, and an outer thread 6 on the widest part of the cylinder, such that it remains anchored onto the cavity 1 a of the lid 12. A handle 5, by way of a gutter, exits from the widest part of the cylinder, where through the slurry may be emptied out.

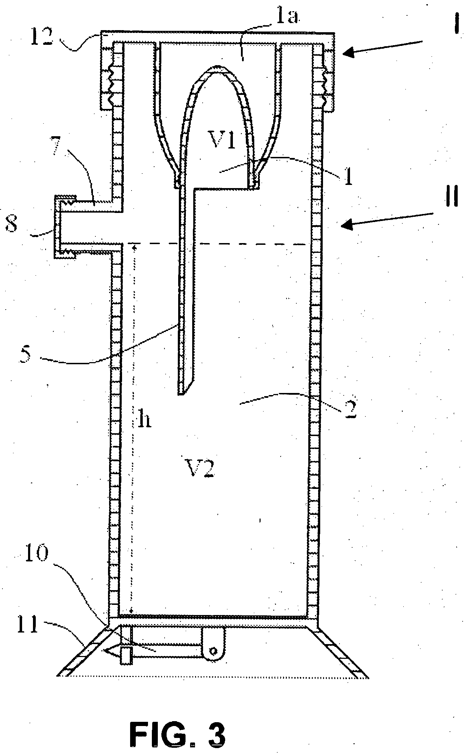

Figure 3 shows a section view of the device for measuring the concentration of ammoniacal nitrogen in slurry of the invention. Said figure 3 shows the first member I and the second member II coupled to one another, said first receptacle 1 being susceptible to being arranged in an inverted position inside the cavity of the second member II upon placing the lid 12. On the base of the outer face thereof, the second member II comprises an anchor arm (element) 10, such that the device may be fixed to the floor in a vertical position in order to ensure the stability thereof. Figure 3 represents a volume V1 of said first receptacle 1 and a volume V2 of said second receptacle 2, where the volume ratio V1/V2 is, in the case of pig slurry, 1 part of slurry / 9 parts of water. The figure also shows that, in addition to volumes V1 and V2, the device houses a volume of air that facilitates performing the mixing by means of vigorous stirring of the slurry and the water in order to prepare the adequate dilution of slurry in the sample to be analyzed, although it should be borne in mind that volume V2 has been represented without including the volume displaced by the handle 5 of the first receptacle 1.

### Description of a preferred embodiment

In order to implement the device according to the invention, the following steps are taken.

The device is placed in a vertical position on a surface that is as horizontal as possible. In the event that it is required, anchor arm 10, which is located on the base of the outer face of the second member II, may be used. This arm guarantees the stability of the device, by contributing to maintaining the vertical position thereof during the taking of water samples in the field, which ensures a precise volume V2.

The first member I is removed by opening the lid. Subsequently, cap 8 is removed from drain 7, which is located on the lateral face of the second member II. The second member II is filled with water until the latter exits through drain 7, the second receptacle 2 housing a volume V2 of water sample in the interior thereof. In order to re-conduct the excess water from the second member II, a tube 9 may be adapted to drain 7. Subsequently, drain 7 is closed with cap 8, after having removed tube 9 if it has been used. Thereafter, the first receptacle 1 for taking slurry samples is unthreaded from cavity 1a of lid 12 of the first member I, and, with the aid of handle 5, a slurry sample with volume V1, defined by the cavity of the first receptacle 1, is collected. The first receptacle 1, now filled with slurry, is threaded to cavity 1 a by means of threads 4 and 6. This configuration of the cavity 1 a with the first receptacle 1 allows for the remains of slurry adhered to the outer surface of the first receptacle 1, once the latter is submerged in the slurry in order to take the slurry sample, not to interfere in the measurement of volume V1, since these remains will be enclosed inside cavity 1 a upon threading the first receptacle 1.

Subsequently, the slurry content with volume V1 is poured, with the aid of the concave channel of handle 5, inside the second member II, and lid 12, which comprises the first receptacle 1, is closed by means of thread 3. Advantageously, all the parts of the device, including the receptacles for taking the slurry samples and the water samples, are integrated in a detachable manner, forming a single piece. The entire ensemble (member I and member II) is stirred in order to prepare a dilution of 100 mL of slurry and 900 mL of water, which is the volume ratio of volumes V1 and V2 in the case of pig slurry. Once the stirring is completed, lid 12 is opened and, with the aid of a conductivity meter with a range between 0-10 dS/m, the conductivity is measured, which corresponds to the concentration of ammoniacal nitrogen in the slurry per cubic meter.

Preferably, the junction means for the different elements that make up the device according to the invention are a thread or similar. However, any other junction means that makes it possible to close the different cubicles for a given period of time will also be adequate.

The general knowledge of persons skilled in the art include replacing each of the parts that make up the device according to the invention with others that have the same function, without this entailing going beyond the scope of protection of the device defined in the attached claims.

Although a specific embodiment of the invention has been explained, it is evident to persons skilled in the art that the manual device designed for measuring ammoniacal nitrogen in slurry or liquid manure is susceptible to numerous variations and modifications, and that all the details mentioned may be replaced with other technically equivalent ones, without going beyond the scope of protection defined by the attached claims.

## Claims

1. Device for measuring the concentration of ammonium nitrogen (NH₄⁺-N) in liquid manure in dilution that comprises a first member (I), which comprises a first receptacle (1) for taking a liquid manure sample with volume V1, and a second member (II), which comprises a second receptacle (2), for taking a water sample with volume V2, in order to prepare a dilution of the sample to be analyzed, **characterized in that** the first member (I) further comprises a closing lid (12), the inner lateral face of said lid (12) includes means (3) designed to join said second member (II) in a detachable manner, and, on the inner face thereof, is equipped with a cavity (1a) designed to house said first receptacle (1) and to enclose inside the cavity (1a) the remains of slurry adhered to the outer surface of said first receptacle (1), when a liquid manure sample of volume V1 is taken with said first receptacle (1), and said cavity (1 a) includes means (4) to join said first receptacle (1) in a detachable manner, allowing said designed cavity (1 a) that the remains of slurry adhered to the outer surface of said first receptacle (1) does not interfere in said volume V1,
**and in that** said two members (I, II), which comprise said two receptacles (1, 2), are configured in such a way that they are susceptible to being coupled to one another in order to obtain said dilution of the sample to be analyzed, where said second member (II) comprises an outflow opening (7) in the lateral face thereof at a height h from the base of said second member (II), wherein said volumes V1 and V2 of said two receptacles (1,2) are sized to provide a dilution whose conductivity measurement fulfils the equivalence 1 dS/m = 1 kg NH₄⁺-N/m³, providing the volume ratio V1/V2 between said two receptacles (1,2) a direct reading of the concentration of ammonium nitrogen per cubic meter of liquid manure from the reading of the conductivity of the dilution prepared.

2. Device according to claim 1, wherein the configurations of said first member (I) and said second member (II) are susceptible to being integrated in a detachable manner such that they form a single piece.

3. Device according to claim 1, wherein said outflow opening (7) delimits the volume V2 of said second receptacle (2).

4. Device according to claim 1, wherein for pig slurry said first receptacle (1) and said second receptacle (2) are adapted to house a volume V1 and a volume V2, respectively, such that the volume ratio V1/V2 is 1 part of liquid manure / 9 parts of water.

5. Device according to claim 1, wherein, when said first member (I) and said second member (II) are coupled to one another, the device further houses a volume of air, such that the liquid manure and the water may be vigorously stirred in order to prepare the dilution of the sample to be analyzed.

6. Device according to claim 1, where said first receptacle (1) is susceptible to being placed in an inverted position inside the cavity of the second member (II) when placing the lid (12).

7. Device according to claim 1, wherein said first receptacle (1) comprises a substantially elongated handle (5) that extends from the upper edge of the cavity of said first receptacle (1), said handle facilitating the taking of liquid manure samples.

8. Device according to claim 7, wherein said substantially elongated handle (5) presents a cross-section with a substantially concave profile, said concave profile defining a channel where through the liquid manure may be emptied out.

9. Device according to claim 1, wherein the outer face of said first receptacle (1) comprises detachable junction means (6) to the cavity (1 a) of the lid (12).

10. Device according to claim 1, wherein said cavity (1 a) of the lid (12) has a conical-trunk shape, the neck of the trunk being adjusted to said first receptacle (1).

11. Device according to claim 1, wherein the detachable junction means (3, 4, 6) are threads or similar.

12. Device according to claim 3, wherein said outflow opening (7) is equipped with closure means (8) or means of conduction (9) for the excess water.

13. Process for analyzing the concentration of ammoniacal nitrogen in liquid manure in the device defined according to any of claims 1 to 12, **characterized in that** it comprises:
- placing the device defined according to any of the preceding claims in a vertical position, optionally with the aid of an anchor element that is located on the outer lower part of the base of the second member;
- removing the first member, which comprises the lid and the first receptacle for taking liquid manure samples, and reserving it;
- removing the closure means of the outflow opening on the lateral face of the second member and filling said second receptacle for taking water samples with water up to the volume V2 defined by the position of said outflow opening, and closing once again the outflow opening with the closure means; subsequently,
- recovering the first member and separating the first receptacle for taking liquid manure samples from the cavity located on the lid and, with the aid of the handle of the first receptacle, taking a sample with volume V1, said volume V1 being defined by the cavity of the first receptacle for taking liquid manure samples;
- joining said first receptacle on said cavity of the lid and, with the aid of the handle, by way of a channel, pouring volume V1 of the liquid manure sample into said second member which contains volume V2 of the water sample, and coupling the first member to the second member;
- vigorously stirring the mixture; and, finally,
- removing the first member and measuring the conductivity of the prepared dilution with a conductivity meter, and
- determining the concentration of ammoniacal nitrogen from a direct reading of the measured conductivity in the prepared dilution, wherein 1 dS/m = 1kg NH₄⁺-N/m³.

14. Kit for measuring the concentration of ammoniacal nitrogen in liquid manure, **characterized in that** it comprises:
- a device as defined in any of claims 1 to 12; and
- a sheet of instructions to prepare the mixture to be analyzed, said sheet of instructions substantially including the steps of the process defined in claim 14 for analyzing the concentration of ammoniacal nitrogen in liquid manure that uses said device.

## Patentansprüche

1. Vorrichtung zum Messen der Konzentration von Ammonium-Stickstoff (NH4⁺-N) in einer flüssigen Jauchelösung mit einem ersten Bauteil (I), welches einen ersten Behälter (1) zum Aufnehmen einer flüssigen Jaucheprobe von einem Volumen V1 aufweist, und einem zweiten Bauteil (II), welches einen zweiten Behälter (2) zum Aufnehmen einer Wasserprobe von einem Volumen V2 aufweist, um eine Lösung der zu analysierenden Probe herzustellen, **dadurch gekennzeichnet, dass** das erste Bauteil (I) zusätzlich einen Verschlussdeckel (12) aufweist, wobei die innere Seitenfläche des Deckels (12) Mittel (3) zum lösbaren Befestigen an dem zweiten Bauteil (II) aufweist, und wobei der Verschlussdeckel (12) an der Innenseite eine Kavität (la) aufweist, die dazu ausgebildet ist, den ersten Behälter (1) aufzunehmen und an der äußeren Oberfläche des ersten Behälters (1) anhaftende Güllereste einzuschließen, wenn eine flüssige Jaucheprobe vom Volumen V1 mit dem ersten Behälter (1) aufgenommen wird, und wobei die Kavität (la) Mittel (4) zum lösbaren Befestigen an dem ersten Behälter (1) aufweist, wobei die derart gestaltete Kavität (la) ermöglicht, dass die Reste von an der äußeren Oberfläche des ersten Behälters (1) anhaftenden Gülleresten nicht in das Volumen V1 eingemischt werden,
**und dadurch gekennzeichnet, dass** die beiden Bauteile (I, II), welche die beiden Behälter (1, 2) aufweisen, derart gestaltet sind, dass diese zur gegenseitigen Kopplung aneinander geeignet sind, um die Lösung der zu analysierenden Probe zu erhalten, wobei das zweite Bauteil (II) in dessen Seitenfläche eine in einer Höhe h vom Boden des zweiten Bauteils (II) aus gelegene Ausflussöffnung (7) aufweist, wobei die Volumen V1 und V2 derart bemessen sind, dass in diesen eine Lösung bereitstellbar ist, deren Leitfähigkeitsmessung die Gleichung 1 dS/m = 1 kg NH₄⁺-N/m³ erfüllt, wobei das Volumenverhältnis V1/V2 zwischen den zwei Behältern (1, 2) ein direktes Bestimmen der Konzentration von Ammonium-Stickstoff pro Kubikmeter flüssiger Jauche aus der Messung der Leitfähigkeit der hergestellten Lösung ermöglicht.

2. Vorrichtung nach Anspruch 1, wobei das erste Bauteil (I) und das zweite Bauteil (II) derart zur gegenseitigen lösbaren Kopplung aneinander geeignet sind, dass diese ein einziges Teil ausbilden.

3. Vorrichtung nach Anspruch 1, wobei die Ausflussöffnung (7) das Volumen V2 des zweiten Behälters (2) begrenzt.

4. Vorrichtung nach Anspruch 1, wobei für Schweinegülle der erste Behälter (1) und der zweite Behälter (2) derart dazu ausgebildet sind jeweils ein Volumen V1 und ein Volumen V2 aufzunehmen, dass das Volumenverhältnis V1/V2 durch 1 Teil flüssige Jauche/9 Teilen Wasser gegeben ist.

5. Vorrichtung nach Anspruch 1, wobei, wenn das erste Bauteil (I) und das zweite Bauteil (II) aneinander gekoppelt sind, in der Vorrichtung zusätzlich ein derartiges Volumen von Luft aufgenommen ist, dass die flüssige Jauche und das Wasser gründlich durchmischbar sind, um die Lösung der zu analysierenden Probe herzustellen.

6. Vorrichtung nach Anspruch 1, wobei der erste Behälter (1) bei Platzierung des Deckels (12) in einer umgekehrten Position innerhalb der Kavität des zweiten Bauteils (II) anordenbar ist.

7. Vorrichtung nach Anspruch 1, wobei der erste Behälter (1) einen im Wesentlichen länglichen Griff (5) aufweist, welcher sich von dem oberen Rand der Kavität des ersten Behälters (1) erstreckt, wobei der Griff die Entnahme von flüssigen Jaucheproben vereinfacht.

8. Vorrichtung nach Anspruch 7, wobei der im Wesentlichen längliche Griff (5) einen Querschnitt mit einem im Wesentlichen konkaven Profil aufweist, wobei das konkave Profil einen Kanal definiert, durch welchen die flüssige Jauche entleerbar ist.

9. Vorrichtung nach Anspruch 1, wobei die Außenfläche des ersten Behälters (1) lösbare Verbindungsmittel (6) zur Ankopplung an die Kavität (la) des Deckels (12) aufweist.

10. Vorrichtung nach Anspruch 1, wobei die Kavität (la) des Deckels (12) eine konische Rumpfform aufweist, wobei das Halsstück des Rumpfs an den ersten Behälter (1) angepasst ist.

11. Vorrichtung nach Anspruch 1, wobei die lösbaren Verbindungsmittel (3, 4, 6) als Gewinde oder dergleichen ausgebildet sind.

12. Vorrichtung nach Anspruch 3, wobei die Ausflussöffnung (7) mit einem Verschlussmittel (8) oder einem Leitungsmittel (9) für das überschüssige Wasser ausgestattet ist.

13. Verfahren zum Analysieren der Konzentration von Ammonium-Stickstoff in flüssiger Jauche in einer Vorrichtung nach einem der Ansprüche 1 bis 12, **dadurch gekennzeichnet, dass** das Verfahren aufweist:
- Anordnen der Vorrichtung nach einem der voranstehenden Ansprüche in einer vertikalen Position, optional mit Hilfe eines Ankerelements, das an dem äußeren unteren Teil des Bodens des zweiten Bauteils angeordnet ist;
- Entfernen des ersten Bauteils, welches den Deckel und den ersten Behälter zum Aufnehmen von Flüssigjaucheproben aufweist, und bereitstellen desselben;
- Entfernen des Verschlussmittels von der Ausflussöffnung an der Seitenfläche des zweiten Bauteils und Füllen des zweiten Behälters zum Aufnehmen von Wasserproben mit Wasser bis zu dem Volumen V2, das durch die Position der Ausflussöffnung definiert ist, und Wieder-Verschließen der Ausflussöffnung mit dem Verschlussmittel; anschließend
- Wiederaufnehmen des ersten Bauteils und Trennen des ersten Behälters zum Aufnehmen von Flüssigjaucheproben von der an dem Deckel angeordneten Kavität und, mit Hilfe des Griffs des ersten Behälters, Aufnehmen einer Probe mit dem Volumen V1, wobei das Volumen V1 durch die Kavität des ersten Behälters zum Aufnehmen von Flüssigjaucheproben definiert ist;
- Verbinden des ersten Behälters mit der Kavität des Deckels und, mit Hilfe des Griffs mittels des Kanals Einfüllen des Volumens V1 der flüssigen Jaucheprobe in das zweite Bauteil, welches das Volumen V2 der Wasserprobe enthält, und Koppeln des ersten Bauteils an das zweite Bauteil;
- gründliches Durchmischen der Mischung;
- Entfernen des ersten Bauteils und Messen der Leitfähigkeit der hergestellten Lösung mit einem Leitfähigkeitsmesser und
- Bestimmen der Konzentration von Ammonium-Stickstoff durchdirektes Ablesen der gemessenen Leitfähigkeit der hergestellten Lösung, wobei 1 dS/m = 1kg NH₄⁺-N/m³.

14. Ausrüstung zur Messung der Konzentration von Ammonium-Stickstoff in einer flüssigen Jauchelösung, **dadurch gekennzeichnet, dass** die Ausrüstung aufweist:
- eine Vorrichtung nach einem der Ansprüche 1 bis 12; und
- eine Gebrauchsanleitung zum Herstellen einer zu analysierenden Mischung, wobei die Gebrauchsanleitung im Wesentlichen die Schritte des Verfahrens gemäß Anspruch 14 zum Analysieren der Konzentration von Ammonium-Stickstoff in flüssiger Jauche unter Benutzung der Vorrichtung enthält.

## Revendications

1. Dispositif de mesure de la concentration de nitrogène d'ammonium (NH₄⁺-N) dans l'engrais liquide en dilution comprenant une première pièce (I), qui comprend un premier réceptacle (1) pour prélever un échantillon d'engrais liquide de volume V1, et une seconde pièce (II), qui comprend un second réceptacle (2), pour prélever un échantillon d'eau de volume V2, afin de préparer une dilution de l'échantillon à analyser, **caractérisé en ce que** la première pièce (I) comprend en outre un couvercle de fermeture (12), la face latérale intérieure dudit couvercle (12) comprenant des moyens (3) conçus pour s'assembler à ladite seconde pièce (II) de manière à pouvoir se détacher, et sa face intérieure étant équipée d'une cavité (1 a) conçue pour accueillir le premier réceptacle (1) et renfermer à l'intérieur de la cavité (1 a) les résidus de purin ayant adhéré à la surface extérieure dudit premier réceptacle (1), lorsqu'un échantillon d'engrais liquide de volume V1 est prélevé à l'aide du premier réceptacle (1), et ladite cavité (1 a) comprend des moyens (4) pour s'assembler audit premier réceptacle (1) de manière à pouvoir se détacher, permettant à ladite cavité (1 a) à la surface extérieure dudit premier réceptacle (1) de laquelle ont adhéré les résidus de purin (1) de ne pas se mêler audit volume V1, **et en ce que** lesdites deux pièces (I,II) comprenant lesdits deux réceptacles (1, 2) sont configurés de telle sorte qu'ils soient susceptibles d'être couplés l'un à l'autre afin d'obtenir ladite dilution de l'échantillon à analyser, où ladite seconde pièce (II) comprend une ouverture d'écoulement (7) sur sa face latérale à une hauteur h de la base de ladite seconde pièce (II), où lesdits volumes V1 et V2 desdits deux réceptacles (1,2) sont dimensionnés pour fournir une dilution dont la mesure de conductivité respecte l'équivalence 1 dS/m = 1 kg NH₄⁺-N/m³, fournissant le rapport de volume V1/V2 entre lesdits deux réceptacles (1, 2) avec lecture directe de la concentration de nitrogène d'ammonium par mètre cube d'engrais liquide à partir de la lecture de la conductivité de la dilution préparée.

2. Dispositif selon la revendication 1, où les configurations de ladite première pièce (I) et de ladite seconde pièce (II) sont susceptibles d'être intégrées de manière à pouvoir se détacher de telle sorte qu'elles forment une seule pièce.

3. Dispositif selon la revendication 1, où ladite ouverture d'écoulement (7) délimite le volume V2 dudit second réceptacle (2).

4. Dispositif selon la revendication 1, où pour le lisier de porc ledit premier réceptacle (1) et ledit second réceptacle (2) sont adaptés pour accueillir un volume V1 et un volume V2, respectivement, de telle sorte que le rapport de volume V1/V2 corresponde à 1 volume d'engrais liquide / 9 volumes d'eau.

5. Dispositif selon la revendication 1, où ladite première pièce (I) et ladite seconde pièce (II) sont couplées l'une à l'autre, le dispositif accueillant en outre un volume d'air, de telle sorte que l'engrais liquide et l'eau puissent être vigoureusement remués afin de préparer la dilution de l'échantillon à analyser.

6. Dispositif selon la revendication 1, où ledit premier réceptacle (1) est susceptible d'être placé en position inversée à l'intérieur de la cavité de la seconde pièce (II) lorsque le couvercle (12) est mis en place.

7. Dispositif selon la revendication 1, où ledit premier réceptacle (1) comprend une poignée considérablement allongée (5) qui s'étend du bord supérieur de la cavité dudit premier réceptacle (1), ladite poignée facilitant le prélèvement des échantillons d'engrais liquide.

8. Dispositif selon la revendication 7, où ladite poignée considérablement allongée (5) présente une vue en coupe avec un profil légèrement concave, ledit profil concave définissant un canal à travers lequel l'engrais liquide peut être vidé.

9. Dispositif selon la revendication 1, où la surface extérieure dudit premier réceptacle (1) comprend des moyens de jonction détachable (6) de la cavité (1 a) du couvercle (12).

10. Dispositif selon la revendication 1, où ladite cavité (1 a) du couvercle (12) possède une forme de tronc conique, le goulot du tronc étant ajusté audit premier réceptacle (1).

11. Dispositif selon la revendication 1, où les moyens de jonction détachable (3, 4, 6) sont des filetages ou similaires.

12. Dispositif selon la revendication 3, où ladite ouverture d'écoulement (7) est équipée de moyens de fermeture (8) ou de moyens de conduction (9) pour l'excès d'eau.

13. Processus d'analyse de la concentration de nitrogène ammoniacal dans l'engrais liquide dans le dispositif défini selon une quelconque des revendications 1 à 12 **caractérisé en ce qu'**il comprend :
- placer le dispositif défini selon une quelconque des revendications précédentes en position vertical, optionnellement à l'aide d'un élément d'ancrage situé sur la partie extérieure basse de la base de la seconde pièce ;
- déposer la première pièce, qui comprend le couvercle et le premier réceptacle de prélèvement d'échantillons d'engrais liquide, et le mettre de côté ;
- déposer les moyens de fermeture de l'ouverture d'écoulement sur la face latérale de la seconde pièce et remplir ledit second réceptacle pour prélèvement d'échantillons d'eau avec de l'eau jusqu'au volume V2 défini par la position de ladite ouverture d'écoulement, et refermer à nouveau l'ouverture d'écoulement avec les moyens de fermeture ; puis,
- recouvrir la première pièce et séparer le premier réceptacle pour prélever des échantillons d'engrais liquide de la cavité située sur le couvercle et, à l'aide de la poignée du premier réceptacle, prélever un échantillon de volume V1, ledit volume V1 étant défini par la cavité du premier réceptacle pour prélever des échantillons d'engrais liquide ;
- joindre ledit premier réceptacle à ladite cavité du couvercle et, à l'aide de la poignée, au moyen d'un canal, verser le volume V1 de l'échantillon d'engrais liquide dans ladite seconde pièce contenant le volume V2 de l'échantillon d'eau, et coupler la première pièce à la seconde pièce ;
- mélanger vigoureusement la mixture ;
- déposer la première pièce et mesurer la conductivité de la dilution préparée avec un mesureur de conductivité, et
- déterminer la concentration de nitrogène ammoniacal à partir d'une lecture directe de la conductivité mesurée dans la dilution préparée, où 1 dS/m = 1 kg NH₄⁺-N/m³.

14. Kit de mesure de la concentration de nitrogène ammoniacal dans l'engrais liquide, **caractérisé en ce qu'**il comprend :
- un dispositif tel que défini dans une quelconque des revendications 1 à 12; est
- une feuille d'instructions pour préparer la mixture à analyser, ladite feuille d'instructions comprenant substantiellement les étapes du processus défini dans la revendication 14 pour analyser la concentration de nitrogène ammoniacal dans l'engrais liquide utilisant ledit dispositif.
